# EUROPEAN PATENT APPLICATION

(11) **EP 4 778 546 A1**
(43) Date of publication of application: **22.07.2026**
(21) Application number: 25841582.7
(22) Date of filing: 18.07.2025
(51) Int. Cl.: A61M 1/36, A61M 1/16, B01D 61/24, B01D 71/16, B01D 71/68, B01D 71/34, B01D 71/40

(54) **BLOOD PERFUSION SYSTEM FOR REMOVING ACTIVE OXYGEN**

(30) Priority: 18.07.2024 KR 20240095254; 18.07.2024 KR 20240095255; 17.07.2025 KR 20250097049
(71) Applicant: Eternox Inc., Seongnam-si, Gyeonggi-do 13605 (KR)
(72) Inventor: JEONG, Han-Gil, Seongnam-si, Gyeonggi-do 13620 (KR); KIM, Jaeyun, Suwon-si, Gyeonggi-do 16419 (KR); IM, Pilseon, Suwon-si, Gyeonggi-do 16419 (KR); KIM, Dong Hee, Osan-si, Gyeonggi-do 18133 (KR)
(74) Representative: V.O.
(86) International application number: PCT/KR2025/010607
(87) International publication number: WO 2026/019291

(57) **Abstract**

The present specification provides: an *in vitro* blood perfusion structure comprising ceria nanoparticles and a support supporting the ceria nanoparticles, whereby reactive oxygen species (ROS) which are in the blood and cause inflammation can be effectively removed *in vitro*; an *in vitro* blood perfusion cartridge and an *in vitro* blood purification device which comprise the structure; a method for manufacturing the structure; and an *in vitro* blood purification method using the *in vitro* blood purification device.

## Description

### [Technical Field]

### [Cross Reference to Related Applications]

This application claims priority to and the benefit of Korean Patent Application No. 10-2024-0095254 filed on July 18, 2024, Korean Patent Application No. 10-2024-0095255 filed on July 18, 2024, and Korean Patent Application No. 10-2025-0097049 filed on July 17, 2025, the disclosure of which is incorporated herein by reference in its entirety.

This specification discloses an *in vitro* blood purification device utilizing ceria nanoparticles (CeNPs) and a support made of various materials capable of supporting the CeNPs, such as supports in the form of porous microbeads, fibers, or membranes, and an *in vitro* blood purification method using the same.

Meanwhile, this application was supported by the following research and development project.

### [R&D Project Supporting the Present Invention]

[Project ID No.] 2022R1C1C100961012
[MSRI Project No.] 08-2023-0123
[Ministry] Ministry of Science and ICT
[Managing Agency] National Research Foundation of Korea
[Program Name] Individual Basic Research (MSIT)
[Project Title] Development of *In vitro* Blood Purification System for Sepsis Treatment Based on Multifunctional Microbeads
[Lead Institution] Seoul National University Bundang Hospital
[Research Period] March 1, 2022 to February 28, 2025

### [R&D Project Supporting the Present Invention]

[Project ID No.] RS-2023-00222910
[MSRI Project No.] 08-2023-0191
[Ministry] Ministry of Science and ICT
[Managing Agency] National Research Foundation of Korea
[Program Name] Bio-Medical Technology Development (R&D)
[Project Title] Development of Technologies for Clinical Application of Five Major Diseases and Fostering Doctor-Scientists through Personalized Future Medicine Research Center for the 6P Medicine Era
[Lead Institution] Seoul National University Bundang Hospital
[Research Period] April 1, 2023 to December 31, 2026

### [R&D Project Supporting the Present Invention]

[Project ID No.] 1711189569
[Sub-project No.] 2022R1A2B5B02002097
[Ministry] Ministry of Science and ICT
[Managing Agency] National Research Foundation of Korea
[Program Name] Individual Basic Research (MSIT)
[Project Title] Development of Nanoparticle-Based Vaccine for Treatment of Multiple Sclerosis Inducing Enhanced Antigen-Specific Immune Tolerance
[Executing Agency] Sungkyunkwan University
[Research Period] March 1, 2023 to February 28, 2025

### [Background Art]

Sepsis is essentially a severe infectious condition accompanied by a systemic inflammatory response. Due to the body's excessive and misguided reaction to infection, sepsis may result in tissue damage and organ dysfunction, potentially progressing to a life-threatening state. Systemic inflammatory response syndrome is a systemic inflammatory response exhibited by the body in response to various forms of severe stress or injury, such as infection, trauma, burns, and pancreatitis, and plays a significant pathophysiological role in sepsis. To date, various drugs have been developed and undergone clinical trials for the treatment of sepsis, but they have failed to demonstrate efficacy. As a new approach to treating sepsis, blood purification therapies (e.g., Toraymyxin, Cytosorb, etc.) designed to remove pathogen-associated substances or cytokines from the body were developed and drew attention. However, despite undergoing clinical trials, the blood purification therapies were unsuccessful, and clinical guidelines currently do not recommend their use. Reactive oxygen species (ROS) are a critical factor in systemic inflammatory response syndrome, including sepsis, where excessive generation or insufficient removal thereof damages cells and promotes inflammation, thereby triggering tissue damage and organ dysfunction. However, existing blood purification therapies have a clear limitation in that they are unable to effectively remove ROS.

Accordingly, after continuous efforts to develop an effective *in vitro* blood perfusion system, the present inventors employed ceria nanoparticles (CeNPs) and further bonded or attached the CeNPs to various materials, particularly polymer-based supports, thereby completing an *in vitro* blood purification device capable of effectively removing ROS from blood and an *in vitro* blood purification method using the same.

### [Disclosure]

### [Technical Problem]

One object of the present invention is to provide an *in vitro* blood perfusion structure using ceria nanoparticles (CeNPs), such as a porous microbead for *in vitro* blood perfusion, a fiber for *in vitro* blood perfusion, and a membrane support for *in vitro* blood perfusion, capable of effectively removing *in vitro* reactive oxygen species (ROS) in the blood that cause inflammation and damage.

Another object of the present invention is to provide an *in vitro* blood perfusion structure, particularly a polymer-based structure, such as a porous microbead for *in vitro* blood perfusion, a fiber for *in vitro* blood perfusion, and a membrane support for *in vitro* blood perfusion, capable of effectively supporting CeNPs and preventing the CeNPs or fragments of the structure containing the CeNPs from detaching from the structure and entering the blood.

Still another object of the present invention is to provide an *in vitro* blood perfusion cartridge including the above-described structure.

Yet another object of the present invention is to provide an *in vitro* blood purification device including the above-described structure.

Yet another object of the present invention is to provide a method of manufacturing the above-described structure.

Yet another object of the present invention is to provide an *in vitro* blood purification method using the above-described *in vitro* blood purification device.

### [Technical Solution]

In order to achieve the above-described objects,

One aspect of the present invention provides a structure for *in vitro* blood perfusion, including ceria nanoparticles (CeNPs) and a support supporting the CeNPs.

In one exemplary embodiment, the support is preferably a polymer-based support, and the polymer is preferably included in an amount of 50% by weight or more based on the total weight of the support.

In one exemplary embodiment, the polymer may be a polymer that may be functionalized to support CeNPs. The support may have a mesoporous or macroporous structure and provide a large surface area for the introduction of a large amount of CeNPs. The support may be functionalized to induce strong electrostatic bonding with the CeNPs by introducing sulfone groups, or to form strong covalent bonds with the CeNPs by introducing amine groups or carboxyl groups. Such physical structure and chemical functionalization allow the CeNPs to be stably supported in large amounts on the support and effectively prevent leaching in a blood perfusion environment.

In one exemplary embodiment, the polymer possesses excellent mechanical strength and biocompatibility in a blood perfusion environment and has properties that allow the polymer to be functionalized to support CeNPs. For example, various polymers described below may be used, and preferably, one or more selected from the group consisting of polystyrene (PS), polyethersulfone (PES), and polyacrylonitrile (PAN) may be included. Specifically, PS is particularly advantageous for CeNPs support and surface modification due to the ease of introducing various functional groups, while PES is characterized by inherent hydrophilicity and excellent biocompatibility. PAN provides excellent chemical resistance and a stable structure.

In one exemplary embodiment, the structure for *in vitro* blood perfusion may be one or more of a porous microbead, a fiber, and a membrane support for *in vitro* blood perfusion.

In one exemplary embodiment, the membrane support may be a dialysis membrane or an ultrafiltration membrane.

In one exemplary embodiment, the structure for *in vitro* blood perfusion may remove reactive oxygen species (ROS) through a catalytic action of CeNPs.

In one exemplary embodiment, the CeNPs may be bonded, attached, or supported on a surface of the support through electrostatic bonding, chemical bonding such as covalent bonding, or a physical adsorption method after surface modification.

In one exemplary embodiment, the structure for *in vitro* blood perfusion may be coated with one or more selected from the group consisting of polyethylene glycol (PEG) and polyvinylpyrrolidone (PVP).

In another aspect, the present invention provides an *in vitro* blood perfusion cartridge including the structure for *in vitro* blood perfusion.

In still another aspect, the present invention provides an *in vitro* blood purification device including the *in vitro* blood perfusion cartridge.

In yet another aspect, the present invention provides an *in vitro* blood purification method including:
(a) a step of bringing blood separated from a subject into contact with the structure for *in vitro* blood perfusion;
(b) a step of removing ROS from the blood using the structure for *in vitro* blood perfusion; and
(c) a step of recovering the blood from which the ROS has been removed.

In yet another aspect, the present invention provides a method of manufacturing the structure for *in vitro* blood perfusion, including:
(a) a step of manufacturing a support capable of supporting CeNPs; and
(b) a step of bonding CeNPs with the structure.

In one exemplary embodiment, the Step (a) may include a step of modifying the surface of the manufactured support.

In one exemplary embodiment, the method may further include a step of coating the surface of the structure for *in vitro* blood perfusion to which CeNPs are attached with a polymer to enhance blood compatibility.

### [Advantageous Effects]

In one aspect, by attaching ceria nanoparticles (CeNPs) to a support capable of supporting CeNPs, preferably a polymer-based support, and more preferably various polymer-based supports that may be functionalized to support CeNPs, such as preferably a porous microbead, a fiber, or a membrane support, the present invention enables the effective removal of reactive oxygen species (ROS) in the blood that cause inflammation and damage. Furthermore, by using a polymer-based support, CeNPs can be effectively supported, and the CeNPs or fragments of the structure including the CeNPs can be prevented from detaching and entering the blood.

Furthermore, an *in vitro* blood purification device configured to circulate a patient's blood through an *in vitro* blood perfusion cartridge including such a structure for *in vitro* blood perfusion can rapidly and extensively remove ROS under both aqueous and actual blood conditions. Therefore, the present invention can be effectively applied to advanced *in vitro* blood perfusion therapy systems.

In another aspect, the present invention enables continuous ROS removal. In particular, to address the problem that continuous removal of ROS is difficult in an *in vitro* blood perfusion environment, the present invention can achieve continuous ROS removal capability by using a catalyst (CeNPs) instead of an adsorbent.

In still another aspect, while inorganic nanoparticles such as CeNPs are difficult to develop into drugs because safety is difficult to guarantee when the inorganic nanoparticles injected into the body, the present invention has the advantage of ensuring safety by removing ROS through blood perfusion outside the body.

In yet another aspect, the present invention can provide the following effects. By maximizing the catalytic properties of CeNPs to rapidly and effectively neutralize excessive ROS in the blood, the present invention can contribute to the treatment of sepsis and various inflammatory diseases. Furthermore, by using a polymer-based flexible support compared to rigid materials such as silica, not only is the loading of the CeNPs more effective but can the CeNPs or fragments of the structure containing the CeNPs be prevented from separating or leaching out and entering the bloodstream. In addition, despite using a polymer support, the support exhibits excellent mechanical properties and durability, allowing the support to maintain stable performance even during prolonged blood perfusion.

In yet another aspect, experimental results acquired by utilizing the structure for *in vitro* blood perfusion of the present invention have demonstrated not only the efficacy of ROS removal but also stability and therapeutic effects in actual blood environments, offering the advantage of high potential for clinical application in sepsis and other inflammatory diseases.

In yet another aspect, unlike conventional *in vitro* perfusion therapies which non-specifically adsorbed even substances beneficial to patients (e.g., anti-inflammatory cytokines), the present invention can selectively remove ROS, which is the fundamental problem of the inflammatory-damaging response, thereby resolving the issues of the conventional *in vitro* blood perfusion devices.

In yet another aspect, , the present invention can remove through *in vitro* perfusion ROS increased in severe diseases related to systemic inflammatory response syndrome, including sepsis, improve severity indicators (e.g., blood pressure, use number of antihypertensive agents), and improve survival rates.

In yet another aspect, the structure for *in vitro* blood perfusion of the present invention can be easily integrated into existing blood perfusion devices and cartridge systems, facilitating application in various treatment environments.

In yet another aspect, the structure for *in vitro* blood perfusion of the present invention can minimize non-specific protein adsorption and thrombus formation upon contact with blood and suppress biological reactivity by coating a surface of the structure with polyvinylpyrrolidone (PVP) or the like.

### [Description of Drawings]

FIGS. 1 to 3 show the results of loading ceria nanoparticles (CeNPs) onto polystyrene/divinylbenzene (PS/DVB) micro beads according to an embodiment of the present invention: FIG. 1 shows a chemical formula illustrating the introduction of sulfonate groups; FIG. 2 is a photograph showing the sulfonation treatment of the PS/DVB micro beads; and FIG. shows a characteristic peak graph illustrating the introduction of sulfonate groups (-SO₃H) in the sulfonated PS/DVB micro beads.
FIGS. 4 to 6 shows optical microscope and scanning electron microscope (SEM) images of micro beads according to an embodiment of the present invention: FIG. 4 shows PS/DVB microbeads; FIG. 5 shows sulfonated PS/DVB microbead (S-PS/DVB); and FIG. 6 shows sulfonated PS/DVB microbeads loaded with ceria (S-PS/DVB@Ce).
FIG. 7 shows an energy dispersive X-ray spectroscopy (EDS) elemental mapping image of an embodiment of the present invention, showing the results of the EDS analysis of S-PS/DVB@Ce microbeads.
FIG. 8 shows SEM images of S-PS/DVB beads that are pre-sulfonated and rich in meso-macro pores in an embodiment of the present invention.
FIG. 9 shows the EDS elemental mapping results of the same beads used in FIG. 8.
FIG. 10 shows SEM images and EDS analysis results of a structure (S-PS fiber@Ce) in which CeNPs are loaded onto a sulfonated polystyrene fiber (S-PS fiber) in an embodiment of the present invention.
FIG. 11 shows optical microscope images illustrating the size change of polyethersulfone (PES)-based microbeads prepared according to an embodiment of the present invention by needle gauge.
FIG. 12 shows optical microscope images illustrating the size change of PES/mesocellular silica foam (MCF) composite microbeads prepared by mixing MCF with PES in an embodiment of the present invention by needle gauge.
FIG. 13 shows the results of quantitatively comparing the average diameters of PES and PES/MCF microbeads prepared using a 20 G needle in an embodiment of the present invention.
FIG. 14 shows schematic diagrams illustrating CeNPs loading and characterization on PES-based microbeads of an embodiment of the present invention.
FIG. 15 shows optical microscope images and SEM images of PES microbeads prepared according to an embodiment of the present invention.
FIG. 16 shows optical microscope images and SEM images of PES@Ce microbeads directly loaded with CeNPs in an embodiment of the present invention.
FIG. 17 shows optical microscope images and SEM images of PES/MCF microbeads prepared including MCF in an embodiment of the present invention.
FIG. 18 shows optical microscope images and SEM images of (PES/MCF)@Ce microbeads prepared by loading CeNPs onto composite beads including MCF in an embodiment of the present invention.
FIG. 19 shows the results of confirming, through a UV-Vis absorbance analysis and a quantitative analysis, the loading of CeNPs onto PES and PES/MCF microbeads in an embodiment of the present invention.
FIG. 20 shows the results of observing a surface of the PES membrane using SEM in an embodiment of the present invention.
FIG. 21 shows the EDS mapping analysis results of PES-membrane@Ce, in which CeNPs are loaded onto a PES membrane in an embodiment of the present invention.
FIG. 22 is a photograph showing the process of electrospinning polyacrylonitrile (PAN) dissolved in dimethylformamide (DMF) into an ethanol/water mixed solvent in an embodiment of the present invention.
FIG. 23 shows SEM images of polyacrylonitrile (PAN) microbeads in an embodiment of the present invention.
FIG. 24 shows surface modification of PAN-based microbeads in an embodiment of the present invention.
FIG. 25 shows a vibrational peak graph confirming the surface modification in an embodiment of the present invention.
FIG. 26 shows the EDS mapping results of PAN-COOH microbeads loaded with CeNPs in an embodiment of the present invention.
FIG. 27 shows the results of comparing the ROS removal performance of PS/DVB-based microbeads in an embodiment of the present invention.
FIG. 28 shows the results of evaluating the ROS removal ability by loading CeNPs onto a porous polystyrene fiber (PS fiber) and a sulfonated polystyrene fiber (S-PS@Ce fiber) in an embodiment of the present invention.
FIG. 29 shows the results of evaluating the ROS removal ability of PES-based microbeads loaded with CeNPs in an embodiment of the present invention.
FIG. 30 shows the results demonstrating the ROS removal persistence when a 0.2 mM hydrogen peroxide (H₂O₂) solution was repeatedly perfused using a CeNPs-loaded microbead-based cartridge in an embodiment of the present invention.
FIG. 31 shows the results of comparing the ROS removal efficiency of a PES membrane and a PES membrane loaded with CeNPs (PES@Ce) in an embodiment of the present invention.
FIG. 32 shows a graph illustrating the results of evaluating the hydrogen peroxide (H₂O₂) removal performance according to the surface modification of PAN-based microbeads and the loading of CeNPs in an embodiment of the present invention.
FIG. 33 shows the results demonstrating that the blood perfusion system of an embodiment of the present invention significantly improved survival rates in an animal model of severe sepsis.
FIG. 34 shows the results demonstrating that the blood perfusion system of an embodiment of the present invention significantly improved hypotension in an animal model of severe sepsis.

### [Modes of the Invention]

Hereinafter, exemplary embodiments of the present invention will be described in detail.

The term "porous" as used herein refers to a structure including mesopores (2 to 50 nm) and macropores (greater than 50 nm) for the effective loading of ceria nanoparticles (CeNPs), and the pores increase the internal or external surface area of a support to expand the contact area with the nanoparticles. In other words, the porous structure can enable CeNPs to come into contact with as many reactive oxygen species (ROS) as possible by providing a large surface area.

In exemplary embodiments of the present invention, CeNPs are used for the structure for *in vitro* blood perfusion. The reason for using CeNPs for the structure for *in vitro* blood perfusion is that CeNPs possess a unique redox cycling capability. Cerium (Ce) is capable of freely switching between oxidation states +3 (Ce³⁺) and +4 (Ce⁴⁺), thereby enabling efficient removal of various ROS, such as superoxides, hydrogen peroxide, hydroxyl radicals, and hypochlorites. In particular, CeNPs exhibit high reactivity through oxygen vacancies on their surface and may serve as an auto-catalytic antioxidant that repeatedly removes ROS. Furthermore, unlike conventional antioxidants, they have the advantage of being able to function stably for a long time without being consumed.

In one aspect, exemplary embodiments of the present invention relate to a structure for *in vitro* blood perfusion including CeNPs and a support supporting the CeNPs.

In one exemplary embodiment, the support may be a porous microbead.

In one exemplary embodiment, the support may be a porous fiber.

In one exemplary embodiment, the support may be a membrane support. The membrane support may be a semi-permeable polymer membrane, such as a dialysis membrane, a high-flow membrane, or an ultrafiltration membrane, and may have, for example, an average pore size of about 5 to 50 nm or a molecular weight cutoff (MWCO) limit of about 10,000 to 100,000 Da. In addition, in one exemplary embodiment, the membrane support may include hollow fiber membrane support or a flat membrane support used in general hemodialysis, high-flow hemodialysis, or continuous renal replacement therapy (CRRT).

In one exemplary embodiment, the support preferably includes a polymer. In particular, the support preferably includes a polymer, specifically in an amount of 50% by weight or more (which may be defined as "polymer-based"). By using a flexible support that includes a polymer, or preferably a polymer-based flexible support, the loading of CeNPs can be made more effective compared to a rigid material such as silica, and the flexible support may be advantageous to prevent CeNPs or fragments of a structure including the CeNPs from separating or leaching out and entering the bloodstream.

In one exemplary embodiment, the support may include a polymer in an amount of 50% by weight or more, 60% by weight or more, 70% by weight or more, 80% by weight or more, 90% by weight or more, or may consist solely of a polymer.

In one exemplary embodiment, the support may be polymer-based (i.e., including a polymer in an amount of 50% by weight or more), and mesocellular silica foam (MCF) may be additionally added.

In one exemplary embodiment, the polymer may be a polymer that may be functionalized to support CeNPs.

For example, the polymer may be chitosan, chitin, polyethylene (PE), polyacrylonitrile (PAN), polyvinylidene fluoride (PVDF), polysulfone (PSU), polyethersulfone (PES), polystyrene (PS), polyvinyl alcohol (PVA), polymethylmethacrylate (PMMA), cellulose, or cellulose acetate.

The chitosan is a natural polymer that possesses excellent biocompatibility and antibacterial activity and may enhance the ROS removal effect. The chitin, which is a precursor of chitosan, provides biocompatibility and biodegradability, and thus is useful for various biomedical applications.

The PE possesses excellent chemical stability and durability and has the advantage of being able to be mass-produced at low cost.

The PAN possesses excellent mechanical strength and chemical resistance and thus may provide a stable structure.

The PVDF possesses high chemical stability and heat resistance, providing the advantage of stable use even under extreme conditions.

The PSU possesses high strength and heat resistance and thus may provide excellent chemical stability.

The PES possesses characteristics similar to PSU and exhibits excellent biocompatibility.

The PS has the advantage of being able to introduce various functional groups, thereby enhancing adsorption capacity.

The PVA offers excellent biocompatibility and water solubility, and has the advantage of being able to be processed into various forms.

The PMMA may offer high transparency and biocompatibility and thus is suitable for medical devices.

The cellulose is a natural material that can provide biocompatibility and environmental friendliness. In addition, the cellulose acetate has the advantage of low cost in addition to biocompatibility.

The various polymer materials described above may be used in porous microbeads, porous fibers, and membrane supports. In particular, the cellulose acetate, PSU, and PES may be preferably used as membrane supports.

In one exemplary embodiment, the support may have a mesoporous or macroporous structure and may provide a large surface area in order to introduce a large amount of CeNPs.

In one exemplary embodiment, the support may be functionalized to induce strong electrostatic bonding with CeNPs by introducing a functional group capable of electrostatic bonding with CeNPs, such as a sulfone group, or to form strong covalent bonds with CeNPs by introducing a functional group capable of forming covalent bonds, such as an amine group or a carboxyl group. Such physical structure and chemical functionalization can stably support CeNPs in large quantities on a support and effectively prevent the separation or leaching of CeNPs or fragments of the structure including the CeNPs in a blood perfusion environment.

In one exemplary embodiment, the polymer may include one or more selected from the group consisting of polystyrene (PS), polyethersulfone (PES), and polyacrylonitrile (PAN), which possess excellent mechanical strength and biocompatibility in a blood perfusion environment and may be functionalized to support CeNPs. As previously described, specifically, PS is particularly advantageous for CeNPs loading and surface modification because PS facilitates the introduction of various functional groups, while PES is characterized by inherent hydrophilicity and excellent biocompatibility. PAN can provide excellent chemical resistance and a stable structure.

In one exemplary embodiment, the structure for *in vitro* blood perfusion may remove ROS through the catalytic action of CeNPs. In other words, the CeNPs exhibit excellent antioxidant performance to continuously neutralize ROS through autocatalytic action.

In one exemplary embodiment, the CeNPs may be bonded, attached, or supported on the surface of the support through chemical bonding, such as electrostatic bonding or covalent bonding, or through physical adsorption after surface modification.

In one exemplary embodiment, the structure for *in vitro* blood perfusion may evenly disperse the CeNPs throughout the support to maximize ROS removal efficiency.

In one exemplary embodiment, the porous microbead may have a size (diameter) of 1 µm or more, 10 µm or more, 100 µm or more, 150 µm or more, 200 µm or more, 250 µm or more, 300 µm or more, 350 µm or more, 400 µm or more, 450 µm or more, or 1500 µm or less, 1400 µm or less, 1300 µm or less, 1200 µm or less, 1100 µm or less, 1000 µm or less, 900 µm or less, 800 µm or less, 700 µm or less, 650 µm or less, 600 µm or less, 550 µm or less, but is not limited thereto. For example, the porous microbead may have a size (diameter) of 1 to 1500 µm, or 10 to 1000 µm, or 100 to 1000 µm. The size (diameter) of the porous microbead may be adjusted considering the size of the cartridge and effective blood perfusion. In addition, in a non-limiting example, the porous microbead may have a size (diameter) larger than a blood cell, for example, greater than 10 µm.

In one exemplary embodiment, the ROS may be one or more selected from the group consisting of superoxide anions, hydrogen peroxide, and hydroxyl radicals, but is not limited thereto.

In one exemplary embodiment, the porous microbead may have a pore volume of 0.1 cm³/g to 13.5 cm³/g, preferably 0.1 cm³/g to 5.0 cm³/g, more preferably 0.5 cm³/g to 1.5 cm³/g, but is not limited thereto. A porous microbead having the above-described pore volume can effectively support CeNPs and remove ROS.

In one exemplary embodiment, the porous microbead may have a surface area of 100 m²/g to 1000 m²/g, preferably 200 m²/g to 900 m²/g, more preferably 300 m²/g to 800 m²/g, but is not limited thereto. A porous microbead having the above-described surface area can effectively support CeNPs and remove ROS.

In one exemplary embodiment, the porous fiber may be a polymer-based fiber or a polymer consisting of a porous fiber with a diameter of about 10 to 50 µm. For example, the porous fiber may be formed of polystyrene (PS), polypropylene (PP), polycaprolactam (PA-6), polyacrylonitrile (PAN), or copolymers thereof, and the surface may be chemically treated for the introduction of highly active functional groups or the loading of nanoparticles. To ensure durability and flow properties, the fiber may have a large surface area and be porous, having a porosity of, for example, about 10% or less, for example, about 0.1% to 10%.

In an exemplary embodiment, the membrane support may be non-porous, but preferably porous. For example, the membrane support may be provided in the form of a hollow fiber membrane or a flat sheet membrane, and may be a semipermeable polymer membrane intended for the removal of specific molecules in blood or plasma. The membrane may generally be formed of polystyrene (PS), polyethersulfone (PES), polyvinylidene fluoride (PVDF), polymethylmethacrylate (PMMA), polyacrylonitrile (PAN), cellulose derivatives, and the like. The physical properties of the membrane may include, for example, an outer diameter of about 200 to 350 µm, an inner diameter of about 150 to 250 µm, a wall thickness of about 30 to 100 µm, a pore size of about 5 to 50 nm, and a porosity of about 30% to 80%. In addition, the MWCO limit may be about 10,000 to 100,000 Da, the permeability coefficient (ultrafiltration (UF) coefficient) may be about 5 to 80 mL/h/mmHg/m², and the surface area may be 0.2 to 2.5 m², which may be adjusted according to the treatment purpose. In addition, the membrane may be surface-modified to improve biocompatibility, such as by coating the membrane with a hydrophilic polymer (e.g., polyvinylpyrrolidone (PVP)), introducing heparin, or introducing functional groups for nanoparticle loading.

In one exemplary embodiment, the structure for *in vitro* blood perfusion may include CeNPs in an amount of 0.5 mg/g or more. Here, 'mg/g' refers to the amount (mg) of CeNPs per 1 g of support.

Specifically, the structure for *in vitro* blood perfusion may include CeNPs in an amount of 0.5 mg/g or more, 1 mg/g or more, 2 mg/g or more, 3 mg/g or more, 4 mg/g or more, 5 mg/g or more, 10 mg/g or more, 15 mg/g or more, 20 mg/g or more, 30 mg/g or more, 40 mg/g or more, or 50 mg/g or more, or may include 500 mg/g or less, 400 mg/g or less, or 300 mg/g or less, but is not limited thereto. For example, a structure for *in vitro* blood perfusion in a cartridge including 100 mg/g or 200 mg/g of CeNPs can effectively perform *in vitro* blood perfusion and ROS removal. In one exemplary embodiment, the structure for *in vitro* blood perfusion may be coated with one or more selected from the group consisting of polyethylene glycol (PEG) and polyvinylpyrrolidone (PVP). By preventing reactivity between the structure for *in vitro* blood perfusion and blood, hemolysis or agglutination can be inhibited, thereby improving the blood compatibility of the structure for *in vitro* blood perfusion. In this regard, a structure for *in vitro* blood perfusion coated with polyethylene glycol (PEG) is preferred, and a structure for *in vitro* blood perfusion coated with polyvinylpyrrolidone (PVP) is more preferred.

In another aspect, the present invention relates to an *in vitro* blood perfusion cartridge including the structure for *in vitro* blood perfusion.

In one exemplary embodiment, the structure for *in vitro* blood perfusion may have a volume ratio of 50% or more, 55% or more, 60% or more, 65% or more, or 70% or more, or 90% or less, 85% or less, or 80% or less based on the entire volume of the *in vitro* blood perfusion cartridge. For example, the structure for *in vitro* blood perfusion may have a volume ratio of 50% to 90% based on the entire volume of the *in vitro* blood perfusion cartridge, but is not limited thereto. *In vitro* blood perfusion and ROS removal can be effectively performed within the above-described volume ratio.

In still another aspect, the present invention relates to an *in vitro* blood purification device including the *in vitro* blood perfusion cartridge.

In yet another aspect, the present invention relates to an *in vitro* blood purification method including:
(a) a step of bringing blood separated from a subject into contact with the structure for *in vitro* blood perfusion;
(b) a step of removing ROS from the blood using the structure for *in vitro* blood perfusion; and
(c) a step of recovering the blood from which the ROS has been removed.

In one exemplary embodiment, the step of removing ROS may be carried out for 2 to 24 hours, but is not limited thereto, and continuous ROS removal is possible by using a catalyst (CeNPs) instead of an adsorbent in the structure for *in vitro* blood perfusion. Therefore, continuous ROS removal is possible for the required time without time constraints.

In yet another aspect, the present invention relates to a method of manufacturing a structure for *in vitro* blood perfusion, including:
(a) manufacturing a support including one or more polymers selected from the group consisting of polystyrene (PS), polyethersulfone (PES), and polyacrylonitrile (PAN); and
(b) bonding CeNPs to the support.

In one exemplary embodiment, Step (a) may include a step of modifying a surface of the manufactured support.

In one exemplary embodiment, Step (a) may modify a surface of the structure for *in vitro* blood perfusion using a formulation including one or more functional groups selected from the group consisting of amine, sulfonic acid, thiol, carboxylic, hydroxyl, and epoxy groups.

In one exemplary embodiment, the method may further include a step of (c) coating the surface of the support to which CeNPs are attached with a polymer.

In one exemplary embodiment, in Step (c), the coating may be performed with one or more selected from the group consisting of polyethylene glycol (PEG) and polyvinylpyrrolidone (PVP), but is not limited thereto.

In one exemplary embodiment, the method may further include a step of coating the surface of the support with heparin and/or a heparin derivative, but is not limited thereto.

Hereinafter, the present invention will be described in more detail through examples. These examples are solely for illustrating the present invention, and it will be obvious to those skilled in the art that the scope of the present invention is not to be interpreted as being limited by these examples.

### [Examples]

### 1. Experimental materials and methods

### (1-1) Ceria nanoparticles (CeNPs)

CeNPs were synthesized by dissolving 6-aminohexanoic acid (6-AHA, 10 mmol) in 60 mL of deionized water (DI water) and heating the resulting solution to 95 °C under magnetic stirring. To this heated solution, 50 mL of a solution of hydrochloric acid (HCl, 70 µL) and cerium (III) nitrate hexahydrate (2.5 mmol) was added. The mixed solution was incubated for one minute. Thereafter, the generated CeNPs were precipitated by mixing with an excess amount of acetone, washed three times with acetone, and dried in a vacuum oven. Finally, the purified CeNPs were dissolved in deionized water, filtered through a 0.2 µm filter, and refrigerated until use.

### (1-2) Preparation of support material, functionalization, and CeNPs loading

### Directly sulfonated polystyrene (PS) microbeads and CeNPs loading

Macroporous polystyrene-divinylbenzene (PS-DVB)-based porous beads were used as the base material. These beads possess sufficient internal or external surface area for effective loading of CeNPs and allow for the introduction or modification of surface functional groups.

Specifically, the representative physicochemical properties of the beads are as follows:

Diameter 300 to 800 µm; pore volume 0.3 mL/g; surface area 950 m²/g, average pore diameter of meso/macro/transport pores 220 Å; and average pore diameter of micropores 15 Å. A sulfonation process was performed by immersing the microbeads in a concentrated sulfuric acid (approx. 95% to 98%) solution at 80 °C for three hours. Thereafter, residual acid was removed through a sufficient washing process with distilled water, and the microbeads were dried.

### Thereafter, CeNPs loading was carried out as described below.

1 g of sulfonated PS-DVB beads were mixed with 40 mL of an aqueous CeNPs solution with a concentration of 5 mg/mL, and CeNPs were loaded by stirring the resulting mixture at room temperature for 24 hours. After loading, non-adsorbed particles were removed by washing five times with deionized water, and the CeNPs-loaded microbeads were dispersed in deionized water and stored under refrigeration.

### Pre-sulfonated PS microbeads and CeNPs loading

In another embodiment, PS-DVB-based porous beads with sulfonic acid functional groups already introduced were used. These beads possess strong acidic cation exchange properties, and CeNPs could be loaded without separate surface treatment.

Specifically, the representative physicochemical properties of the beads are as follows: diameter 300 to 1200 µm; pore volume 0.3 mL/g; surface area 660 m²/g; average pore diameter of meso/macro/transport pores 650 Å; and average pore diameter of micropores 15 Å.

The CeNPs loading process was performed under the same conditions as above (5 mg/mL CeNPs aqueous solution, stirring for 24 hours, and refrigerated storage after washing five times).

### PS fiber and CeNPs loading

Commercially available porous PS fibers or (PS/DVB) fibers were used as the base material. The fibers possess sufficient internal or external surface area for effective support of CeNPs and allow for the introduction or modification of surface functional groups.

Specifically, the porous fibers may have a pore volume of 0.3 mL/g more, a surface area of 600 m²/g or more, and an average pore diameter of 500 Å or more. The fibers underwent a sulfonation process to introduce negatively charged sulfone groups to the surface of the fibers. The sulfonation process was performed by immersing the fibers in a concentrated sulfuric acid (approx. 95% to 98%) solution at 80 °C for three hours. Thereafter, residual acid was removed through a sufficient deionized water washing process, and the fibers were dried. Thereafter, CeNPs loading was performed as described below.

Thereafter, the loading of CeNPs was performed as follows.

The sulfonized polystyrene fibers were cut into 100 mm x 100 mm pieces, and 40 mL of CeNPs with a concentration of 5 mg/mL was mixed per fiber piece. The resulting mixture was then stirred at room temperature for 24 hours to load the CeNPs. After loading, non-adsorbed CeNPs were removed by washing with deionized water five times for 30 minutes each time, moisture was removed using ethanol, and then the CeNPs were dried under vacuum. Thereafter, the sulfonated polystyrene fibers loaded with CeNPs were stored under refrigeration.

### Polyethersulfone (PES)-based microbeads and CeNPs loading

### (1) PES microbeads and CeNPs loading

PES microbeads were prepared by electrospinning a 10% by weight PES solution (solvent: dimethylformamide (DMF)) into a 1:1 ethanol/water mixture (electrospinning voltage 7 kV, PES solution flow rate 0.5 mL/min).

10 mL of the prepared PES beads were mixed with 40 mL of a 5 mg/mL CeNPs aqueous solution, and the resulting mixture was stirred at room temperature for 24 hours to load the beads with CeNPs.

Thereafter, the beads were washed five times with deionized water, dispersed in deionized water, and refrigerated until use.

### (2) PES/mesocellular silica foam (MCF) composite microbeads and CeNPs loading

To assist in the loading of CeNPs, 1 g of MCF was dispersed in a 10% by weight PES solution (DMF, 20 mL) and then electrospun into a 1:1 ethanol/water mixture to prepare PES/MCF composite microbeads (Electrosion voltage 7 kV, PES solution flow rate 0.5 mL/min)

The prepared PES/MCF beads (10 mL) were mixed with an aqueous CeNPs solution (5 mg/mL, 40 mL) under the same conditions as above, and loading was performed for 24 hours. After washing five times, the CeNPs-loaded microbeads were dispersed in deionized water and stored in a refrigerator.

### PES dialysis membrane and CeNPs loading

In this example, a flat membrane support made of PES was used as a semipermeable polymer membrane. The membrane had specifications of a diameter of 25 mm, a pore size of approximately 30 nm, and a thickness of 110 µm, and a commercial membrane product with a flat-sheet structure was purchased from Surface Engineered Membrane (SEM) and used.

As a structure having characteristics essentially similar to a general hemodialysis membrane in terms of structural form, pore size, and materials used, this membrane was utilized as an exemplary model for implementing a dialysis membrane to evaluate the feasibility of loading CeNPs according to the present invention.

40 mL of CeNPs at a concentration of 5 mg/mL was mixed, and the resulting mixture was stirred for loading at room temperature for 24 hours. After loading, non-adsorbed CeNPs were removed by washing with deionized water five times for 30 minutes each time, moisture was removed using ethanol, and the CeNPs-loaded membrane was dried under vacuum.

### Polyacrylonitrile (PAN) microbeads and CeNPs loading

Porous microbeads were directly prepared using a PAN polymer (e.g., average molecular weight M approx. 150,000). PAN microbeads were prepared by electrospinning a 3% by weight PAN solution (solvent: DMF) into a 1:1 ethanol/water mixture (electrospinning voltage 7 kV, PAN solution flow rate 0.5 mL/min). To modify the surface to be negatively charged with the carboxyl groups, the prepared PAN beads (7.5 mL) were dehydrated and then mixed with 40 mL of a 1 M NaOH solution, and the resulting mixture was stirred for 24 hours. Thereafter, the beads were washed with deionized water five times for 30 minutes each time. The PAN beads, negatively charged with the carboxyl groups, exhibited an orange coloration.

To load CeNPs on PAN beads surface-modified with carboxyl groups, 10 mL of the prepared beads were mixed with 40 mL of CeNPs at 5 mg/mL, loaded with the CeNPs for 24 hours, and after washing 5 times, the CeNPs-loaded beads were dispersed in deionized water and stored in the refrigerator.

### (1-3) Coating process (polyvinylpyrrolidone (PVP))

The microbeads, fibers, and membranes loaded with CeNPs were coated with PVP to improve blood compatibility and prevent the detaching of CeNPs.

### Coating with PVP

The microbeads, fibers, or membranes loaded with CeNPs (e.g., 1 g) were mixed with a 5 mg/mL PVP solution (20 mL) for six hours, then washed with deionized water and dried in a vacuum oven. The resulting P-Ce-polymer supports (microbeads, fibers, or membranes) were refrigerated until use.

### (1-4) Blood perfusion cartridge fabrication and system configuration

A cartridge case included an upper portion, a barrel portion, and a lower portion. Each portion of the cartridge case was designed using Fusion 360 software (Autodesk, CA, USA) and fabricated using an SLA-based ProJet 7000 3D printer (3D Systems, SC, USA). Accura ClearVue was used as the resin to visualize the inside of the cartridge. Silicone O-rings were inserted into the connections between the portions of the cartridge case to prevent leakage. The inside of the assembled cartridge case was filled with 200 mg of blood perfusion microbeads, and then he blood perfusion cartridge was assembled.

### (1-5) Sepsis animal experiments

The experimental groups included a group treated with P-Ce-PS/DVB (ceria-loaded and PVP-coated polystyrene-divinylbenzene microbeads) and an untreated group that received only standard treatment. Male Sprague-Dawley rats aged 9 to 14 weeks and weighing 300 to 450 g were used. For anesthesia induction, the rats were sedated initially with a 4% isoflurane, followed by intramuscular injection of tiletamine/zolazepam (30 mg/kg) and xylazine (10 mg/kg). Subsequently, endotracheal intubation was performed using a 16-gauge catheter, and mechanical ventilation was performed using a mechanical ventilator (Inspira Advanced Safety Ventilator). Sedation was maintained with 0.5% to 1% isoflurane connected to the ventilation circuit, and tramadol (500 mcg) was administered subcutaneously for analgesia.

Under aseptic conditions, cannula were inserted into the left common carotid artery, right common femoral artery, and left common femoral vein using 24-gauge catheters. Blood pressure was monitored throughout the experiment via a pressure transducer and monitor connected to the arterial lines. Body temperature was monitored with a rectal probe and regulated with an infrared heater to be maintained between 36.5 °C and 37.5°C. All fluids and medications were administered through a 3-way stopcock connected to the left femoral vein catheter. The blood perfusion device circuit started at the left common carotid artery cannula, connected via tubing to a blood perfusion cartridge, followed by additional tubing to a 3-way stopcock for infusion and sampling, and finally connected to the left common femoral vein catheter. The circuit was initially filled with 100 mL of heparinized saline (50 IU/mL) and subsequently washed with 100 mL of saline. To induce refractory septic shock, 5 mg/kg of lipopolysaccharide (LPS) derived from *E. coli* O111:B4 was intravenously administered for 10 minutes. Subsequently, 30 mL/kg of normal saline was infused via fluid resuscitation for 10 minutes. Blood perfusion was initiated using a peristaltic pump, and blood was drawn via the left common carotid artery cannula at a flow rate of 5 mL/kg/min. The blood was perfused through the cartridge and returned to the left common femoral vein. When the mean arterial pressure (MAP) was less than 60 mmHg, norepinephrine infusion at 0.1 meg/kg/min was initiated, and the rate could be adjusted up to a maximum of 2 mcg/kg/min depending on the MAP.

Differences in survival curves between groups were evaluated using the log-rank (Mantel-Cox) test. Changes in blood pressure between groups over time were analyzed using a mixed-effects model that considered both fixed effects (treatment group) and random effects (individual variability).

### 2. Experimental results

### (2-1) Analysis of characteristics of manufactured microbeads, fibers, and membranes

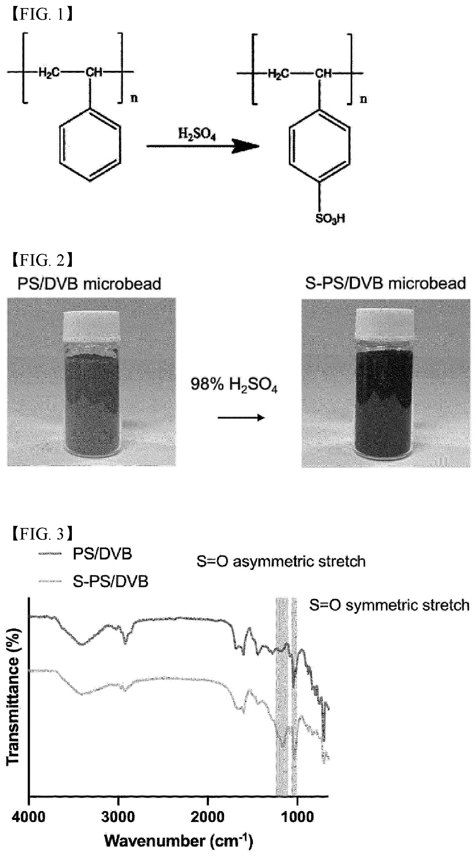

FIGS. 1 to 3 show the results of PS/DVB microbead - CeNPs loading according to an embodiment of the present invention: FIG. 1 shows a chemical formula illustrating the introduction of sulfonate groups; FIG. 2 is a photograph showing the sulfonation treatment of the PS/DVB micro beads; and FIG. 3 shows a characteristic peak graph illustrating the introduction of sulfonate groups (-SO₃H) in the sulfonated PS/DVB micro beads.

As can be seen in FIG. 3, the results of the Fourier transform infrared spectroscopy (FTIR) spectral analysis confirmed that characteristic peaks (S=O asymmetric/symmetric stretching vibrations, in the range of 1000 cm⁻¹ to 1200 cm⁻¹) representing the introduction of sulfonate groups (-SO₃H) in sulfonated PS/DVB microbeads successfully appeared. These results demonstrate that the surface functionalization (sulfonation) of PS/DVB microbeads was effectively achieved.

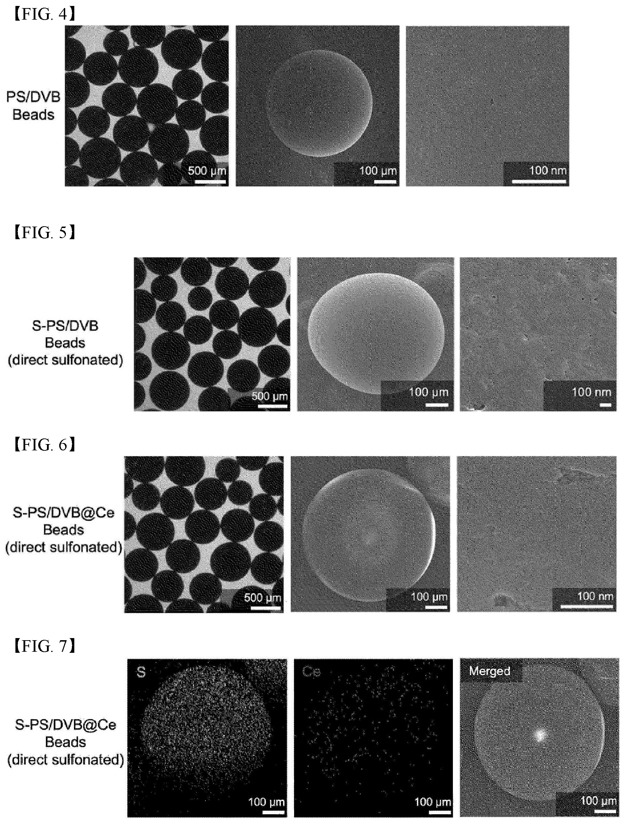

FIGS. 4 to 6 shows optical microscope and scanning electron microscope (SEM) images of micro beads according to an embodiment of the present invention: FIG. 4 shows PS/DVB microbeads; FIG. 5 shows sulfonated PS/DVB microbead (S-PS/DVB); and FIG. 6 shows sulfonated PS/DVB microbeads loaded with ceria (S-PS/DVB@Ce). The images show that the uniform spherical shape of the microbeads was maintained even after sulfonation and CeNPs loading, and that CeNPs were evenly attached to the surface.

FIG. 7 shows an energy dispersive X-ray spectroscopy (EDS) elemental mapping image of an embodiment of the present invention, showing the results of the EDS analysis of S-PS/DVB@Ce microbeads. As can be seen in FIG. 7, the sulfur (S) element mapping (left) confirmed that sulfur was widely distributed on the surface of the sulfonated polymer. The cerium (Ce) element mapping (middle) clearly confirmed that CeNPs (Ce) were effectively loaded onto the microbead surface and evenly distributed. These results strongly demonstrate that sulfonation was successful and that positively charged CeNPs were stably attached to the introduced sulfonic acid functional groups through electrostatic attraction.

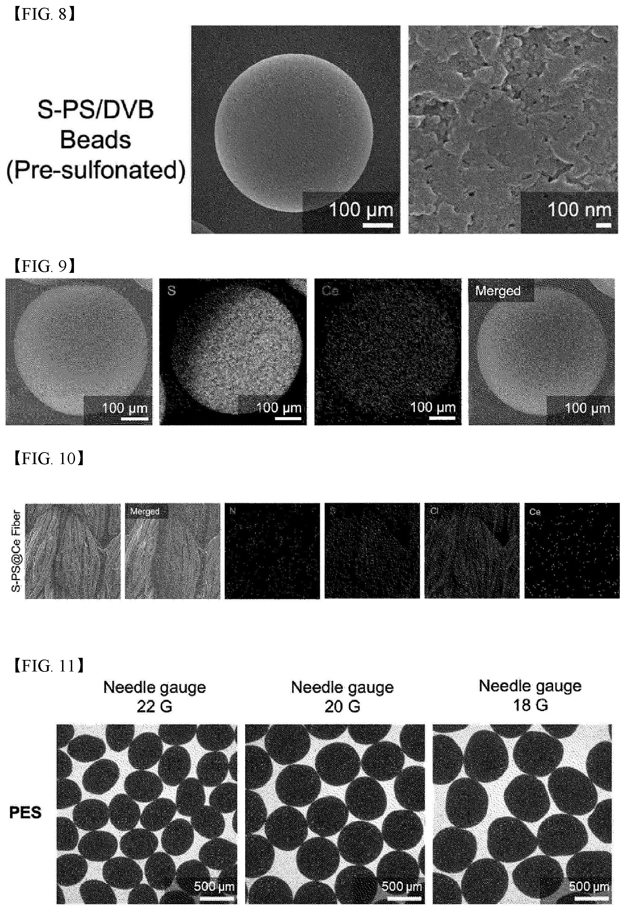

FIG. 8 shows SEM images of S-PS/DVB beads that are pre-sulfonated and rich in meso-macro pores.

Surface (left): Beads with a diameter ranging from 300 to 1200 µm maintained a smooth appearance, while fine irregularities and porous patterns were observed.

Cross-section (right): A thickly intertwined internal framework and a continuous meso-macro pore network were identified, reflecting beads having an average pore diameter increased to approximately 650 Å. This structure enhances the penetration and loading capacity of CeNPs.

FIG. 9 shows the EDS elemental mapping results of the same beads used in FIG. 8.

As can be seen in FIG. 9, the sulfur (S) signal was uniformly distributed throughout the fibrous backbone, indicating that sulfonic acid groups (-SO₃H) were already present throughout the beads.

In addition, the cerium (Ce) signal was also evenly distributed throughout the interior, proving that the CeNPs were stably loaded across the entire surface of the beads and within the pores. The simultaneous distribution of both sulfur and cerium strongly suggests that the nanoparticles were completely immobilized due to electrostatic interactions between the negatively charged sulfonyl groups and the positively charged CeNPs.

FIG. 10 shows SEM images and EDS analysis results of a structure (S-PS fiber@Ce) in which CeNPs are loaded onto a sulfonated polystyrene fiber (S-PS fiber). The SEM images confirm that the fiber structure is well formed, and the EDS mapping results confirm that ceria (Ce) is uniformly distributed across the fiber surface. In addition, the presence of the S element supports the introduction of sulfonate groups, while the distribution of the Ce element supports ceria loading.

FIG. 11 shows optical microscope images illustrating the size change of polyethersulfone (PES)-based microbeads prepared according to an embodiment of the present invention by needle gauge. As the needle gauge was changed to 22 G, 20 G, and 18 G, the average diameter of the microbeads showed a gradual increase, and in particular, when using an 18 G needle, the diameter was largest (approximately 600 to 800 µm). Under each condition, the microbeads maintained uniform sphericity.

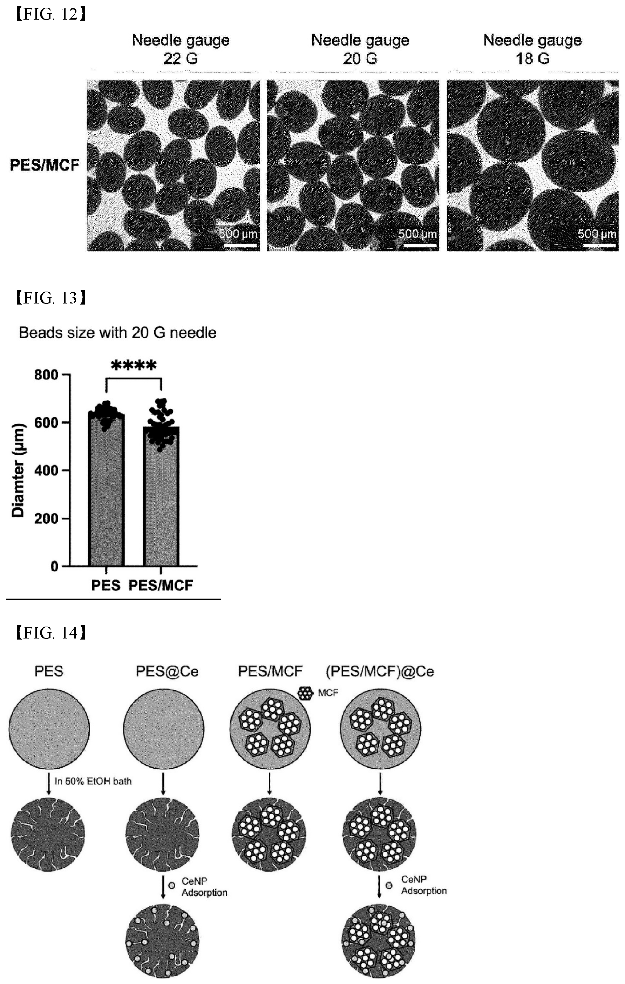

FIG. 12 shows optical microscope images illustrating the size change of PES/MCF composite microbeads prepared by mixing MCF with PES in an embodiment of the present invention by needle gauge. Similar to the beads made of the PES polymer alone, the size of the microbeads tended to increase as the needle gauge increased, while maintaining excellent shape stability and sphericity overall.

FIG. 13 shows the results of quantitatively comparing the average diameters of PES and PES/MCF microbeads prepared using a 20 G needle. The PES/MCF composite microbeads exhibited a significantly smaller average diameter compared to the beads made of the PES polymer alone (****, p < 0.0001), which demonstrates that changes in the composition of polymer composites may affect the physical behavior of the microbead formation process.

FIG. 14 shows schematic diagrams illustrating CeNPs loading and characterization on PES-based microbeads of an embodiment of the present invention. The first schematic diagram on the left illustrates the basic PES microbeads prepared by electrospinning a 10% by weight PES solution (in DMF) into a 50% ethanol/water mixed solution. The second schematic diagram illustrates a structure (PES@Ce) in which CeNPs are physically adsorbed onto a surface of the beads by immersing the PES microbeads in an aqueous CeNPs solution. The third schematic diagram illustrates the composite microbeads (PES/MCF) prepared by electrospinning a mixture of MCF and a PES solution, and the fourth schematic diagram illustrates a (PES/MCF)@Ce structure manufactured by adsorbing CeNPs onto the composite beads. The MCF internally includes a porous silica structure, which serves to increase the loading capacity of CeNPs.

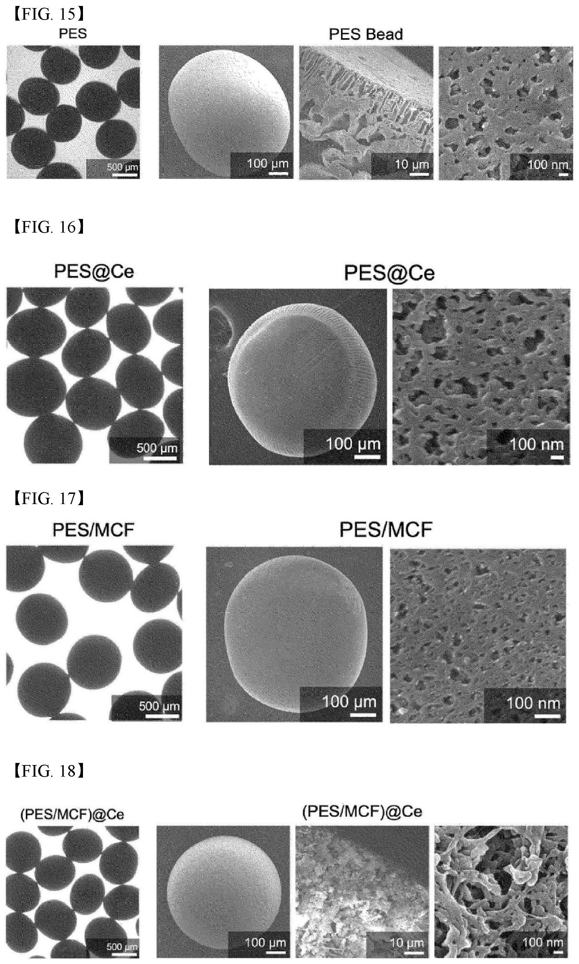

FIG. 15 shows optical microscope images and SEM images of PES microbeads prepared according to an embodiment of the present invention. As confirmed in the optical images, the microbeads prepared from the PES polymer alone maintained a uniform and precise spherical shape, with an average diameter of approximately 600 µm. The SEM analysis results confirmed a smooth outer surface, an internal cross-section with a spongy porous structure, and a structure with uniformly distributed nano-sized pores. The PES microbeads were used as a control for future loading experiments.

FIG. 16 shows optical microscope images and SEM images of PES@Ce microbeads directly loaded with CeNPs in an embodiment of the present invention. The optical microscope images confirmed that the PES@Ce microbeads maintained a spherical structure similar to the beads made of PES alone, while the SEM analysis showed fine attachment of the CeNPs to the surface. In addition, the internal cross-section showed that the nanoporous structure was maintained, suggesting that ceria was evenly dispersed within the matrix without aggregation.

FIG. 17 shows optical microscope images and SEM images of PES/MCF microbeads prepared including MCF in an embodiment of the present invention. The optical microscope images show that the sphericity and size were similar to the beads made of the PES polymer alone, while the SEM analysis confirm the formation of a denser and more complex porous structure due to the influence of MCF introduced into the PES matrix. This structure enables the simultaneous achievement of improved surface area and structural reinforcement of the beads.

FIG. 18 shows optical microscope images and SEM images of (PES/MCF)@Ce microbeads prepared by loading CeNPs onto composite beads including MCF in an embodiment of the present invention. The optical microscope images confirmed excellent morphological stability, and the SEM analysis results showed that the ceria particles are uniformly attached to the surface. The internal cross-section confirmed that ceria was effectively loaded within the MCF structure, and therefore the porous channel structure was maintained while the ceria were present in an integrated form within the network structure.

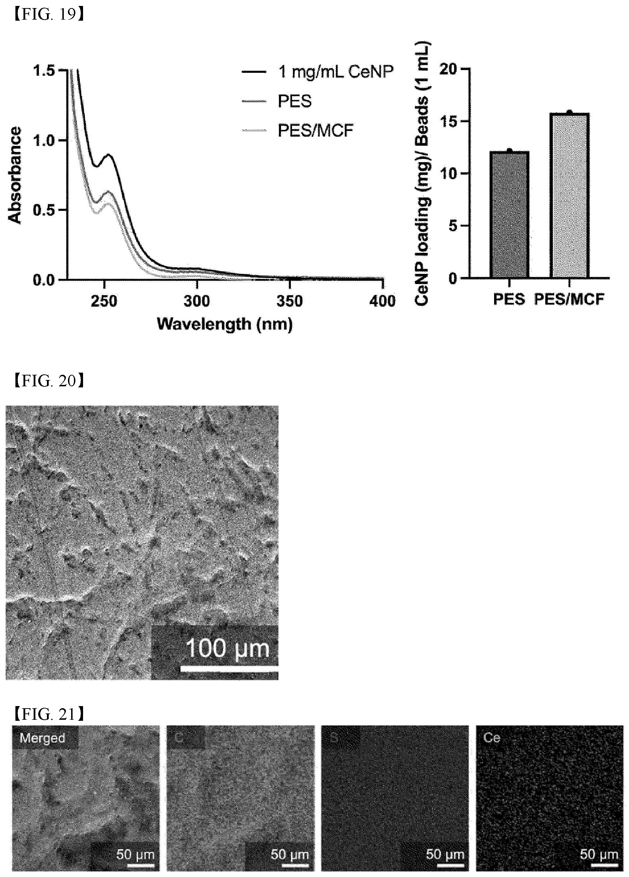

FIG. 19 shows the results of confirming, through a UV-Vis absorbance analysis and a quantitative analysis, the loading of CeNPs onto PES and PES/MCF microbeads in an embodiment of the present invention. In the left panel, a CeNP-specific absorption peak (around 260 nm) was observed in both PES and PES/MCF microbeads, demonstrating successful CeNPs loading. The quantitative analysis shown in the right panel confirmed that the PES/MCF microbeads loaded with more CeNPs than the PES microbeads based on the same volume (1 mL).

FIG. 20 shows the results of observing a surface of the PES membrane using SEM in an embodiment of the present invention. The fine uneven structure and porous surface observed in the SEM image demonstrate the unique physical properties of the membrane, and the structure is interpreted as a suitable base structure for surface modification and functionalization for CeNPs loading.

FIG. 21 shows the EDS mapping analysis results of PES-membrane@Ce, in which CeNPs are loaded onto a PES membrane in an embodiment of the present invention. The analysis results confirmed that the Ce signal was uniformly distributed throughout the membrane, and these results qualitatively suggest that the CeNPs were effectively loaded onto the surface. These characteristics support the idea that the membrane of the present embodiment can serve as a basis for implementing ROS removal functions.

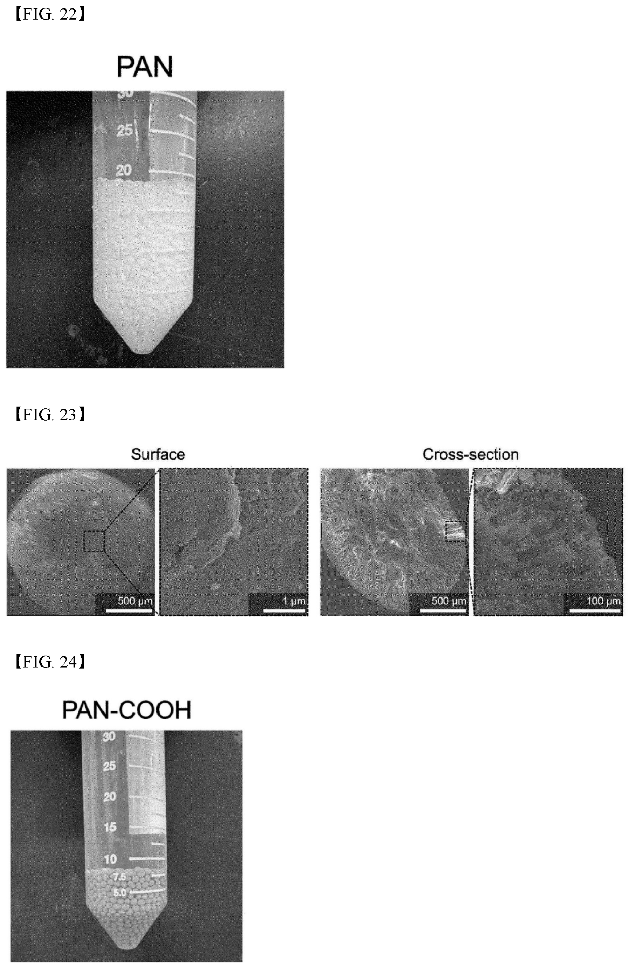

FIG. 22 shows that in an embodiment of the present invention, during the process of electrospinning PAN dissolved in DMF into an ethanol/water mixed solvent, the internal DMF was rapidly removed by solvent exchange with the external solvent, and insoluble PAN was precipitated in the form of beads. As a result, white PAN beads were formed.

FIG. 23 shows SEM images of PAN microbeads in an embodiment of the present invention, which respectively show the surface and cross-sectional structures of the microbeads. The SEM images of the surface on the left show that the PAN microbeads maintained a generally uniform spherical shape, and the high-magnification image show a fine porous surface structure. The cross-sectional images on the right confirm a porous channel structure unevenly distributed throughout the interior, which indicates that pores were formed inside the microbead as well.

These surface and internal structures provide a high specific surface area and diffusion pathways for the effective loading of functional nanomaterials such as CeNPs, thereby serving as an advantageous base structure for implementing ROS removal functions during *in vitro* blood perfusion.

FIG. 24 shows surface modification of PAN-based microbeads in an embodiment of the present invention. PAN is a polymer that inherently include nitrile groups (-CN), and modification to a negatively charged surface is necessary for the loading of CeNPs. Accordingly, the PAN microbeads were treated with a basic aqueous solution to hydrolyze the nitrile groups, converting them into carboxyl groups (-COOH), and through this process, the negative charge on the microbead surface increased. After the treatment, a change in the color of the microbeads from white to orange was visually confirmed, indicating that the surface modification was successfully achieved.

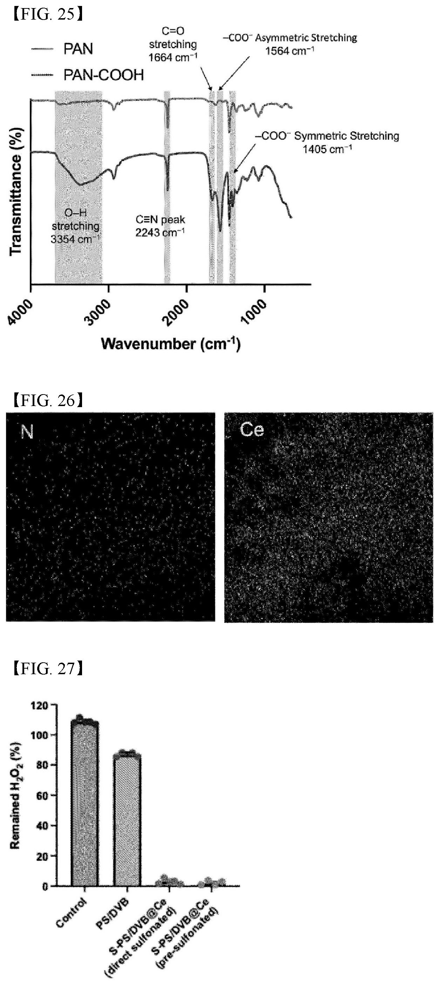

FIG. 25 shows that the conventional PAN exhibited a vibrational peak at 2243 cm⁻¹ due to the nitrile functional group, and in the surface-modified PAN-COOH, a broad peak appeared at 3354 cm⁻¹ due to the O-H vibrational absorption of the carboxyl group. In addition, the typical C=O peak of the carboxyl group appeared at 1664 cm⁻¹, and the symmetric and asymmetric peaks of the negatively charged COO⁻ appeared at 1405 and 1564 cm⁻¹, confirming that the PAN-COOH was surface-modified with carboxyl groups.

FIG. 26 shows the EDS mapping results of PAN-COOH microbeads loaded with CeNPs in an embodiment of the present invention. Signals of nitrogen (N) and cerium (Ce) elements were distributed throughout the surface of the microbeads, suggesting that ceria nanoparticles were effectively loaded.

FIG. 27 shows the results of comparing the ROS removal performance of PS/DVB-based microbeads in an embodiment of the present invention. The control group and PS/DVB microbeads without ceria loading showed almost no effect on H₂O₂ removal, whereas the ceria-loaded S-PS/DVB@Ce microbeads significantly reduced H₂O₂ concentration. In particular, excellent ROS removal ability was confirmed in both the direct sulfonation method and the pre-sulfonation methods, demonstrating that the CeNPs were effectively functionalized under both conditions.

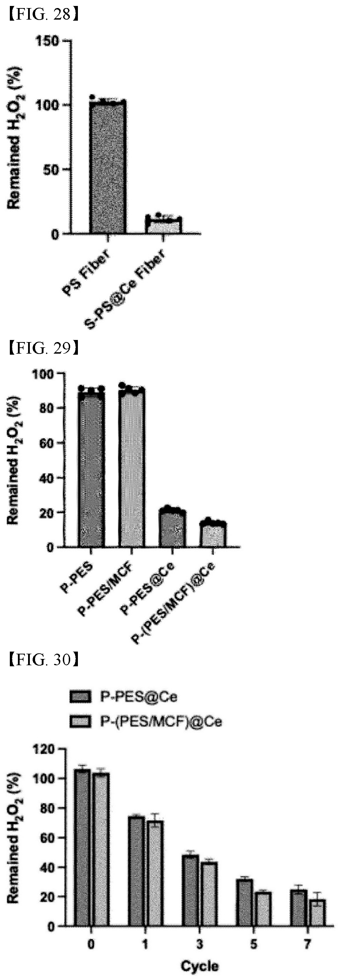

FIG. 28 shows the results of evaluating the ROS removal ability by loading CeNPs onto a porous PS fiber and a sulfonated polystyrene fiber (S-PS@Ce fiber) in an embodiment of the present invention. The PS fiber without CeNPs loading exhibited almost no ROS removal efficacy, whereas the sulfonated PS fiber loaded with ceria exhibited an ROS removal efficiency of about 90%.

FIG. 29 shows the results of evaluating the ROS removal ability of PES-based microbeads loaded with CeNPs in an embodiment of the present invention. As can be seen in FIG. 29, the control groups without ceria (PES and PES/MCF) exhibited almost no ROS removal effect, whereas the ceria-loaded PES@Ce and (PES/MCF)@Ce microbeads removed about 80% of hydrogen peroxide (H₂O₂), exhibiting significant ROS removal ability.

FIG. 30 shows the results demonstrating the ROS removal persistence when a 0.2 mM hydrogen peroxide (H₂O₂) solution was repeatedly perfused using a CeNPs-loaded microbead-based cartridge in an embodiment of the present invention. A cartridge filled with 1 mL of the microbeads was repeatedly perfused with 10 mL of H₂O₂ solution at a rate of 1 mL/min, and the residual H₂O₂ concentration was measured at each cycle (0, 1, 3, 5, 7). The results showed that both PES@Ce and PES/MCF@Ce microbeads stably maintained the H₂O₂ removal ability even with repeated perfusion, and in particular, PES/MCF@Ce exhibited slightly lower residual H₂O₂ concentrations overall, indicating slightly superior ROS removal efficiency. These results demonstrate that the ceria nanoparticles act catalytically to maintain ROS removal activity even after repeated use.

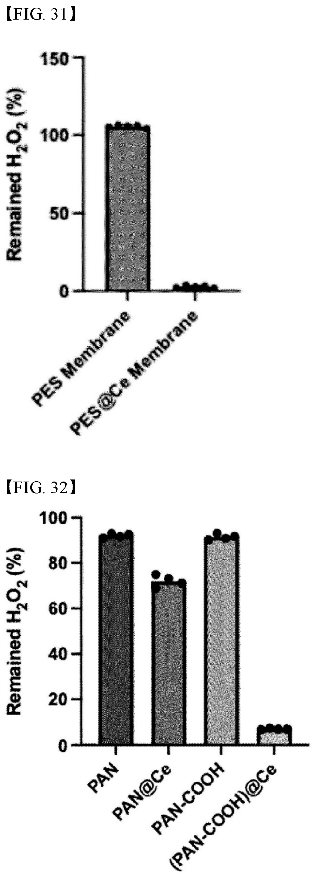

FIG. 31 shows the results of comparing the ROS removal efficiency of a PES membrane and a PES membrane loaded with CeNPs (PES@Ce) in an embodiment of the present invention.

While the PES membrane without ceria loading exhibited almost no ability to remove hydrogen peroxide (H₂O₂), the PES@Ce membrane removed about 95% of H₂O₂, demonstrating excellent ROS removal efficiency. These results suggest that the porous structure of the PES membrane enables effective loading of CeNPs, and that ceria-loaded membranes can be effectively utilized as materials for ROS removal.

FIG. 32 shows a graph illustrating the results of evaluating the hydrogen peroxide (H₂O₂) removal performance according to the surface modification of PAN-based microbeads and the loading of CeNPs in an embodiment of the present invention.

Here, the experimental group consists of four conditions, each classified as follows:
(1) PAN: Pure PAN beads without modification or loading;
(2) PAN@Ce: PAN beads with directly loaded CeNPs;
(3) PAN-COOH: PAN beads surface-modified with carboxyl groups (-COOH); and
(4) (PAN-COOH)@Ce: CeNPs-loaded PAN beads surface-modified with carboxyl groups.

FIG. 32 shows that the loading of CeNPs contributes to H₂O₂ removal, and that the H₂O₂ removal ability is maximized, particularly when CeNPs are loaded after the PAN surface is modified with carboxyl groups. These results demonstrate that carboxyl group modification induces stable loading of CeNPs, thereby significantly improving the efficiency of ROS removal.

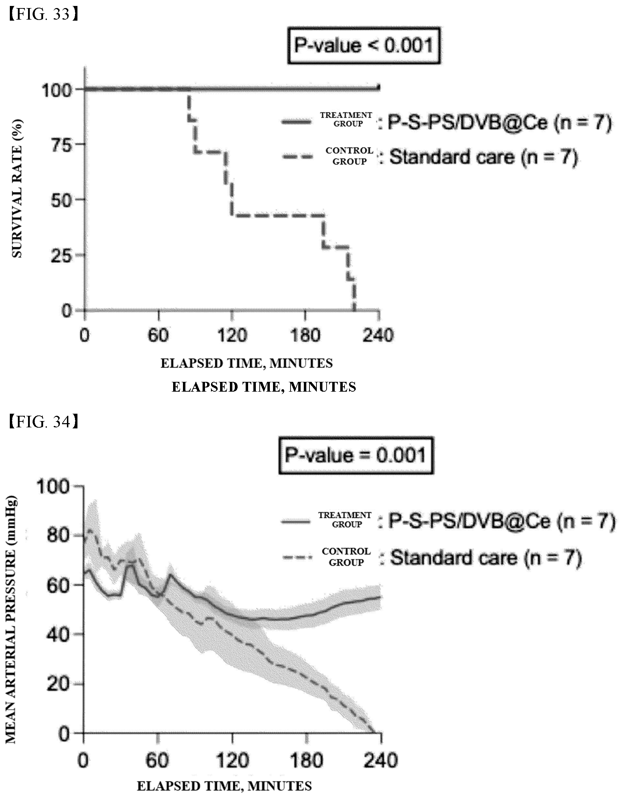

FIG. 33 shows the results demonstrating that the blood perfusion system of an embodiment of the present invention significantly improved survival rates in an animal model of severe sepsis.

In this experiment, the microbeads (P-S-PS-DVB@Ce) loaded with ceria nanoparticles (CeNPs) on sulfonated PS-DVB and surface-coated with PVP were used for blood perfusion. The group treated with this system showed a survival rate of 100%, which was significantly different from the 0% survival rate of the control group that received only the standard treatment. These results clearly demonstrate that the S-PS-DVB@Ce-based blood perfusion system of the present invention can effectively reduce mortality due to sepsis.

FIG. 34 shows the results demonstrating that the blood perfusion system of an embodiment of the present invention significantly improved hypotension in an animal model of severe sepsis. The X-axis represents the elapsed time after lipopolysaccharide (LPS) administration, and the Y-axis represents the mean arterial pressure (MAP, mmHg). The control group (the group receiving only the standard treatment, dotted line) showed a continuous decrease in mean arterial pressure, which is a typical pattern of refractory septic shock, whereas the group treated with the blood perfusion system of an embodiment of the present invention (e.g., perfusion using PVP-coated S-PS/DVB@Ce microbeads, solid line) showed a tendency that the MAP was maintained at a constant level or increased, demonstrating that the hypotensive condition was effectively improved.

### [Industrial Applicability]

The present invention relates to an *in vitro* blood perfusion system capable of contributing to the treatment of sepsis or various inflammatory diseases by effectively removing reactive oxygen species (ROS) in the blood that cause inflammation outside the body.

## Claims

1. A structure for *in vitro* blood perfusion, comprising ceria nanoparticles and a support supporting the ceria nanoparticles.

2. The structure for *in vitro* blood perfusion of claim 1, wherein the support is a polymer-based support in which a polymer is included in an amount of 50% by weight or more based on the total weight of the support.

3. The structure for *in vitro* blood perfusion of claim 1, wherein the support includes one or more polymers selected from the group consisting of chitosan, chitin, polyethylene, polyacrylonitrile (PAN), polyvinylidenefluoride, polysulfone, polyethersulfone (PES), polystyrene (PS), polyvinyl alcohol, polymethylmethacrylate, cellulose, and cellulose acetate.

4. The structure for *in vitro* blood perfusion of claim 1, wherein the support includes one or more polymers selected from the group consisting of polystyrene (PS), polyethersulfone (PES), and polyacrylonitrile (PAN).

5. The structure for *in vitro* blood perfusion of claim 1, wherein the support is a porous microbead.

6. The structure for *in vitro* blood perfusion of claim 1, wherein the support is a porous fiber.

7. The structure for *in vitro* blood perfusion of claim 1, wherein the support is a membrane support.

8. The structure for *in vitro* blood perfusion of claim 1, wherein the *in vitro* blood perfusion structure removes reactive oxygen species through a catalytic action of the ceria nanoparticles.

9. The structure for *in vitro* blood perfusion of claim 5, wherein the porous microbead has a size (diameter) of 1 to 1500 µm.

10. The structure for *in vitro* blood perfusion of claim 5, wherein the porous microbead has a pore volume of 0.1 cm³/g to 13.6 cm³/g.

11. The structure for *in vitro* blood perfusion of claim 5, wherein the porous microbead has a surface area of 100 m²/g to 1,000 m²/g.

12. The structure for *in vitro* blood perfusion of claim 6, wherein the porous fiber has a diameter of 10 to 50 µm.

13. The structure for *in vitro* blood perfusion of claim 6, wherein the porous fiber has a porosity of 10% or less.

14. The structure for *in vitro* blood perfusion of claim 7, wherein the membrane support has an outer diameter of 200 to 350 µm, an inner diameter of 150 to 250 µm, a wall thickness of 30 to 100 µm, a pore size of 5 to 50 nm, and a porosity of 30% to 80%.

15. The structure for *in vitro* blood perfusion of claim 7, wherein the membrane support has a molecular weight cutoff (MWCO) limit of 10,000 to 100,000 Da, a ultrafiltration (UF) coefficient of 5 to 80 mL/h/mmHg/m², and a surface area of 0.2 to 2.5 m².

16. The structure for *in vitro* blood perfusion of claim 1, wherein the structure for *in vitro* blood perfusion includes the ceria nanoparticles in an amount of 0.5 mg/g or more.

17. The structure for *in vitro* blood perfusion of claim 1, wherein the structure for *in vitro* blood perfusion is coated with one or more selected from the group consisting of polyethylene glycol (PEG) and polyvinylpyrrolidone (PVP).

18. An *in vitro* blood perfusion cartridge comprising the structure for *in vitro* blood perfusion of any one of claims 1 to 17.

19. The *in vitro* blood perfusion cartridge of claim 18, wherein the structure for *in vitro* blood perfusion has a volume ratio of 50% to 90% based the total volume of the *in vitro* blood perfusion cartridge.

20. An *in vitro* blood purification device comprising the *in vitro* blood perfusion cartridge of claim 18.

21. An *in vitro* blood purification method comprising:
(a) a step of bringing blood separated from a subject into contact with the structure for *in vitro* blood perfusion of any one of claims 1 to 17;
(b) a step of removing reactive oxygen species from the blood using the structure for *in vitro* blood perfusion; and
(c) a step of recovering the blood from which the reactive oxygen species has been removed.

22. A method of manufacturing the structure for *in vitro* blood perfusion of any one of claims 1 to 17, comprising:
(a) a step of manufacturing a structure for *in vitro* blood perfusion, including one or more polymers;
(b) a step of combining ceria nanoparticles with the structure for *in vitro* blood perfusion.

23. The method of claim 22, wherein Step (a) includes a step of modifying a surface of the structure for *in vitro* blood perfusion using a formulation including one or more functional groups selected from the group consisting of amine, sulfonic acid, thiol, carboxylic, hydroxyl, and epoxy groups.

24. The method of claim 22, further comprising a step of coating a surface of the structure for *in vitro* blood perfusion with one or more polymers selected from the group consisting of polyethylene glycol (PEG) and polyvinylpyrrolidone (PVP).
